# EUROPÄISCHE PATENTSCHRIFT

(11) **EP 2 228 456 B1**
(45) Veröffentlichungstag und Bekanntmachung des Hinweises auf die Patenterteilung: **23.11.2011**
(21) Anmeldenummer: 10155226.3
(22) Anmeldetag: 02.03.2010
(51) Int. Cl.: C12Q 1/68

(54) **Selektion kodierender Nukleinsäure-Konstrukte auf Abwesenheit von Frameshift-Mutationen**
Selection of encoding nucleic acid constructs for absence of frameshift mutations
Sélection de modèles d'acides nucléiques à codage sur la présence de mutations frame-shift

(30) Priorität: 02.03.2009 DE 102009011253
(43) Veröffentlichungstag der Anmeldung: 15.09.2010
(73) Patentinhaber: GENEART AG, 93053 Regensburg (DE)
(72) Erfinder: Liss, Michael, 93059 Regensburg (DE); Notka, Frank, 93059 Regensburg (DE); Daubert, Daniela, 84028 Landshut (DE); Benkel, Claudia, 93047 Regensburg (DE)
(74) Vertreter: Wöhler, Christian

(56) Entgegenhaltungen:
- EP-A1- 0 872 560
- EP-A1- 1 275 736
- WO-A1-2008/077881
- WO-A1-2009/056343
- WO-A2-02/06527
- WO-A2-2008/051619
- DE-B3-102007 052 344
- JP-A- 2004 242 583
- US-B1- 6 391 641
- OHASHI-KUNIHIRO S ET AL: "A novel vector for positive selection of inserts harboring an open reading frame by translational coupling" BIOTECHNIQUES, Bd. 43, Nr. 6, Dezember 2007 (2007-12), Seiten 751-754, XP002584556 EATON PUBLISHING COMPANY US
- CABANTOUS STÉPHANIE ET AL: "New molecular reporters for rapid protein folding assays." PLOS ONE, Bd. 3, Nr. 6, 2008, Seite E2387, XP002584557 ISSN: 1932-6203

## Beschreibung

### Beschreibung

Die vorliegende Erfindung betrifft ein Verfahren zur Bestimmung von Frameshift-Mutationen in kodierenden Nukleinsäuresequenzen.

Genetische Information ist in kodierenden Nukleinsäuren in Form von Basentriplets oder Kodons organisiert. Bei der Gensynthese treten jedoch häufig Fehler in Form unterschiedlicher Arten von Mutationen auf. Bei den Mutationen handelt es sich großteils um Einzelbasendeletionen und Insertionen (ca. 90 %). Im Gegensatz zu den meisten Substitutionen führen nahezu alle Insertionen und Deletionen zu so genannten Frameshifts. Geht eine Base eines Gens im Rahmen einer Mutation verloren (Deletion) oder wird eine Base hinzugefügt (Insertion), so verändert sich das Leseraster der nachfolgenden Basentriplets. Eine Frameshift-Mutation hat biologisch wesentlich größere Auswirkungen als eine Substitution. Ab der Insertion/Deletion werden andere Aminosäuren kodiert und es kommt meist zu einem Abbruch der Translation durch out-of-frame Stoppkodons.

Mit zunehmender Länge eines synthetisierten Gens steigt die Wahrscheinlichkeit für das Auftreten einer Mutation stark an. Der Großteil der ungewollten Mutationen kommt durch das Fehlen oder die Insertion von Einzelbasen in Oligonukleotiden zustande. Die Wahrscheinlichkeit einer Substitution einer Base liegt bei der Synthese von Genen oder Genbibliotheken bei etwa 0,1-0,2 ‰ pro Position, die Wahrscheinlichkeit für eine Insertion/Deletion liegt bei 1,0-1,5 ‰ pro Position. Beispielsweise beobachteten Kong et al., Nucleic Acid Res. (2007), 35:e61, bei der Chipbasierten Gensynthese eine Deletionsrate von 1,48 ‰ pro Position und eine Substitutionsrate von 0,3 ‰ pro Position. Etwa die Hälfte der Substitutionen kommt wiederum durch PCR-Fehler zustande.

Die bei Insertionen und Deletionen auftretende Verschiebung des Leserasters kann genutzt werden, um über einen Reportervektor solche Nukleinsäure-Fragmente zu selektieren, deren Leseraster intakt ist. So offenbart die europäische Patentanmeldung EP 0 872 560 A1 ein Verfahren zur Bestimmung von Frameshift-Mutationen, bei dem mittels homologer Rekombination ein Konstrukt erzeugt wird, das einen Promotor, ein zu untersuchendes Gen und im gleichen Leseraster mit dem zu untersuchenden Gen ein Reportergen enthält. Bei der Expression des Leserasters wird ein Fusionsprotein erhalten, welches die Reportergenfunktion beinhaltet. Dieses kann über phänotypische Eigenschaften nachgewiesen werden.

Problematisch ist allerdings, dass das zu untersuchende Gen für die in EP 0 872 560 A1 beschriebene Vorgehensweise keine internen Stoppkodons innerhalb des offenen Leserasters (ORF) aufweisen darf. Dies ist jedoch bei vielen synthetisierten Genen der Fall.

Angesichts der obigen Problematik und aufgrund der Notwendigkeit, auch für Nukleinsäuren, welche interne Stoppkodons umfassen, ungewollte Leserastermutationen zu detektieren bzw. auszuselektieren, wurde in dem deutschen Patent DE 10 2007 052 344 B3 ein Verfahren zur Bestimmung von Frameshift-Mutationen in kodierenden Zielnukleinsäuren offenbart, welches die Schritte umfasst:
(i) Bereitstellen einer Wirtszelle, umfassend eine doppelsträngige Nukleinsäure, welche eine kodierende Zielnukleinsäure und eine dazu komplementäre, kodierende Gegenstrangnukleinsäure umfasst, worin die Gegenstrangnukleinsäure in operativer Verknüpfung mit einem Reportergen in 3' Position vorliegt;
(ii) Bewirken der Expression der Gegenstrangnukleinsäure; und
(iii) Bestimmen, ob in der Wirtszelle eine Expression des Reportergens erfolgt,
   worin die Expression des Reportergens anzeigt, dass die Zielnukleinsäure keine Frameshift-Mutation aufweist.

Der Begriff "keine Frameshift-Mutation" ist dabei so zu verstehen, dass die Zielnukleinsäure entweder überhaupt keine Frameshift-Mutation aufweist oder dass in der Zielnukleinsäure mehrere Mutationen vorliegen, die für sich betrachtet, zu einer Verschiebung des Leserahmens führen würden, die sich aber gegenseitig wieder aufheben. Beispielsweise kann in der Zielnukleinsäure eine Kombination von Insertionen und Deletionen vorliegen, die sich aber gegenseitig wieder aufheben, so dass keine Änderung des Leserasters auftritt und das Reportergen weiter korrekt abgelesen werden kann.

Das Prinzip der DE 10 2007 052 344 B3 besteht darin, dass für die Bestimmung von Frameshift-Mutationen anstelle der kodierenden Zielnukleinsäure eine dazu komplementäre kodierende Gegenstrangnukleinsäure genutzt werden kann.

Der Begriff "kodierend" ist in diesem Zusammenhang so zu verstehen, dass die Gegenstrangnukleinsäure so beschaffen ist, dass sie die Expression des 3' verknüpften Reportergens ermöglicht. Die Gegenstrangnukleinsäure muss zu diesem Zweck aber nicht unbedingt durch einen offenen Leserahmen definiert sein.

Bei der Durchführung des Verfahrens wird zunächst eine Wirtszelle bereitgestellt, die eine doppelsträngige Nukleinsäure umfasst, welche eine kodierende Zielnukleinsäure und eine dazu komplementäre, kodierende Gegenstrangnukleinsäure enthält. Bei der Zielnukleinsäure handelt es sich vorzugsweise um eine synthetisch erzeugte Sequenz. Die Gegenstrangnukleinsäure ist in der Wirtszelle operativ mit einem Reportergen verknüpft, welches sich stromabwärts in 3'-Position befindet.

Wenn die Gegenstrangnukleinsäure intakt ist, d.h. keine Mutationen wie insbesondere Insertionen oder Deletionen aufweist, die eine Änderung des Leserasters zur Folge haben, so wird bei der Expression ein Fusionspolypeptid erhalten, das die von der Gegenstrangnukleinsäure kodierte Aminosäuresequenz sowie C-terminal davon die Aminosäuresequenz des von dem Reportergen kodierten Produkts aufweist. Wenn die Gegenstrangnukleinsäure dagegen eine Frameshift-Mutation aufweist, d.h. insbesondere eine Insertion oder Deletion, durch die sich das Leseraster ändert, so führt diese Verschiebung des Leserasters dazu, dass das Reportergen nicht in-frame zu der kodierenden Gegenstrangnukleinsäure liegt. Folglich wird das Reportergen nicht exprimiert. Der Reporter wird also nur dann exprimiert, wenn das Leseraster des Gegenstrangs intakt ist.

Wenn die Gegenstrangnukleinsäure keine Frameshift-Mutationen aufweist, so kann daraus geschlossen werden, dass auch die komplementäre Zielnukleinsäure selbst intakt ist und keine Frameshift-Mutationen aufweist.

Das Verfahren der DE 10 2007 052 344 B3 beruht darauf, dass nur bei Abwesenheit von Frameshift-Mutationen die Translation des Gegenstrangs zu einem Fusionsprotein führt, welches den Reporter umfasst. Unter Umständen weist ein solches Fusionsprotein jedoch aufgrund seiner Größe ein hohes Molekulargewicht auf und kann in Form von denaturierten Aggregaten in der Zelle vorliegen. Die Expression und Funktionalität des Reporters kann also auch bei Einfügen von korrekten Leserahmen durch die Eigenschaften des gebildeten Polypeptids beeinflusst oder sogar inhibiert werden. Dies kann insbesondere bei relativ großen kodierenden Zielnukleinsäuren und entsprechenden Gegenstrangnukleinsäuren zu einer Verringerung der Effizienz des Verfahrens führen.

Angesichts dieser Problematik wurde die in DE 10 2007 052 344 B3 offenbarte Vorgehensweise von den Erfindern weiterentwickelt. Überraschenderweise wurde herausgefunden, dass die im Fachbereich bekannte Strategie der translationellen Kopplung in dem Verfahren der DE 10 2007 052 344 B3 zur operativen Verknüpfung der kodierenden Gegenstrangnukleinsäure mit einem Reportergen in 3'-Position genutzt werden kann.

Das Phänomen der konjugierten Translation oder der translationellen Kopplung ist ein Regulierungsmechanismus, bei dem die Translation eines stromaufwärtigen Gens die Translation eines stromabwärtigen Gens reguliert. Eine Theorie zur Erklärung dieses Phänoms geht davon aus, dass das Ribosom von dem translatierten stromaufwärtigen Gen an das stromabwärtige Gen über ein Translationskopplungssignal übergeben wird, welches als schwache Ribosomenbindungsstelle fungiert. Dabei erfolgt keine erneute Ribosomenanbindung, sondern das Ribosom kann nach Beendigung der Translation des stromaufwärts gelegenen Gens die Sequenz scannen und die Translation des stromabwärtigen Gens auslösen. Die translationelle Kopplung zwischen zwei Cistrons wird also über dasselbe Ribosom vermittelt. Das Ribosom beendet die Translation an einem Stoppkodon in der stromaufwärts gelegenen Sequenz und scannt daraufhin die stromabwärtige Sequenz, wobei es bei einem Startkodon in der Nähe dieses Stoppkodons mit der erneuten Translation beginnt. Dieser Scannvorgang des Ribosoms erfolgt in beide Richtungen, so dass die Fortsetzung der Synthese an einem Startkodon ausgelöst werden kann, welches mit dem Stoppkodon der vorhergehenden kodierenden Sequenz überlappt.

Die translationelle Kopplung wurde erstmals von Oppenheim 1980 für das Tryptophan-Operon in *E. coli* beschrieben (D.S. Oppenheim und C. Yanowski (1980), Genetics 95:789-795). Der Effekt beruht darauf, dass die Translation nach einem Stoppkodon bei einem direkt darauf folgenden Startkodon wieder einsetzt. Dabei entstehen zwei getrennte Polypeptidketten. Voraussetzung für die Synthese des zweiten Polypeptids ist korrektes Ablesen des ersten Leserahmens bis hin zum Stoppkodon.

US 6391641 B1 beschreibt ein Verfahren zur Auswahl von Missense-Mutanten unter Verwendung einer "upstream" gelegenen Sequenz eines Zielgens und eines "downstream" gelegenen Reportergens, die translationell gekoppelt sind.

WO 2008/077881 A1 beschreibt ein Verfahren zur Selektion von Genen einer Genbibliothek auf eine verbesserte Expressionsleistung, wobei die verbesserte Expression mittels eines Reportergens quantifiziert wird, welches durch translationelle Kopplung mit dem entsprechenden Gen synthetisiert wird. Gleichzeitig werden durch dieses Verfahren Gene mit offenem Leserahmen identifiziert. Als Reporter werden z.B. Resistenzgene oder GFP genannt. Es wird zudem darauf hingewiesen, dass das Startkodon des Reportergens sowohl mit dem Stoppkodon des offenen Leserahmens überlappen als auch einen Abstand von bis zu 500 Nukleotiden aufweisen kann.

WO 2008/051619 A1 beschreibt ein Verfahren zum Screening von DNA-Bibliotheken zur Identifikation von DNA-Fragmenten mit offenem Leserahmen, die weder interne ribosomale Bindestellen noch interne Stoppkodons aufweisen. Zur Selektion der Sequenzen werden Reporterproteine verwendet, die nicht durch kovalente Fusion mit dem entsprechenden Leserahmen, sondern durch translationelle Kopplung synthetisiert werden, um eine Fehlfaltung bzw. Fehlfunktion eines entsprechenden Fusionsproteins zu verhindern. Das Dokument offenbart zudem zwei Vektorvarianten, die entweder eine positive Selektion von offenen Leserahmen ohne Stoppkodons über Resistenzmarker ermöglichen, oder eine negative Selektion von offenen Leserahmen mit internen ribosomalen Bindestellen über Expression eines Toxins bewirken.

Ohashi-Kunihiro et al. (Biotechniques (2007) 43(6):741-2, 754) beschreiben ebenfalls die Selektion von DNA-Fragmenten mit offenem Leserahmen durch translationelle Kopplung mit einem Resistenzmarker, der nur in Abwesenheit von internen Stoppkodons exprimiert wird. Zudem wird der optimale Abstand des offenen Leserahmens zu dem Resistenzmarker ermittelt.

Cabantous et al. (PLoS one 3(6): e2387) beschreibt ein GFP-Reportersystem, welches dazu geeignet ist eine Frameshift-Mutation in einer kodierenden Zielnukleinsäure anzuzeigen, wobei GFP im Falle einer Frameshift-Mutante durch translationale Kopplung exprimiert wird.

In der vorliegenden Erfindung wurde nun überraschenderweise herausgefunden, dass die Strategie der translationellen Kopplung in dem in DE 10 2007 052 344 B3 offenbarten Verfahren zur Selektion kodierender Nukleinsäurekonstrukte auf Abwesenheit von Frameshift-Mutationen genutzt werden kann. Im Gegensatz zu den im Stand der Technik beschriebenen Verfahren wird im erfindungsgemäßen Verfahren jedoch der offene Leserahmen des Gegenstrangs der kodierenden Zielnukleinsäure durch translationelle Kopplung selektioniert.

Die Erfindung stellt daher ein Verfahren zur Bestimmung von Frameshift-Mutationen in kodierenden Zielnukleinsäuren bereit, welches die Schritte umfasst:
(i) Bereitstellen einer Wirtszelle, umfassend eine doppelsträngige Nukleinsäure, welche eine kodierende Zielnukleinsäure und eine dazu komplementäre kodierende Gegenstrangnukleinsäure umfasst, worin die Gegenstrangnukleinsäure über einen Linker mit einem Reportergen in 3'-Position verknüpft ist;
(ii) Bewirken der Expression der Gegenstrangnukleinsäure; und
(iii) Bestimmen, ob in der Wirtszelle eine Expression des Reportergens erfolgt,
   worin die Expression des Reportergens anzeigt, dass die Zielnukleinsäure keine Frameshift-Mutation aufweist,
   dadurch gekennzeichnet,
   dass der Linker eine translationelle Kopplersequenz aufweist, die ein Stoppkodon *in frame* zu dem Leserahmen der Gegenstrangnukleinsäure und ein Startkodon umfasst, wobei das Reportergen *in frame* zu dem Startkodon liegt.

Wenn die Gegenstrangnukleinsäure (und damit auch die komplementäre Zielnukleinsäure) keine Frameshift-Mutation aufweist, so ist ein korrektes Ablesen des Gegenstrangs bis hin zu dem Stoppkodon der translationellen Kopplersequenz in dem in 3'-Position angebundenen Linker möglich. An dem darauf folgenden Startkodon der translationellen Kopplersequenz setzt dann die Translation wieder ein. Da das Startkodon in dem erfindungsgemäß eingesetzten Linker *in frame* zu einem darauf folgenden Reportergen liegt, wird in diesem Fall auch das Reportergen translatiert.

Durch die Nutzung der translationellen Kopplung in dem Verfahren der vorliegenden Erfindung entstehen somit das Expressionsprodukt der zu überprüfenden Gegenstrangnukleinsäure und des Reportergens als getrennte Polypeptidketten.

Mit Hilfe des erfindungsgemäßen Verfahrens gelingt es, selbst bei Vorliegen von internen Stoppkodons in einer Zielnukleinsäure Frameshift-Mutationen unter Nutzung der Gegenstrangnukleinsäure mittels eines Reportergens zu bestimmen. Interne Stoppkodons der Zielnukleinsäure werden bei der Nutzung des Leserasters im Gegenstrang als Leu bzw. Ser translatiert. Die Nutzung eines Leserasters im Gegenstrang hat weiterhin den Vorteil, dass eine mögliche Toxizität des von der eigentlichen Zielnukleinsäure kodierten Proteins für die Wirtszelle nicht relevant ist, da nur der Gegenstrang translatiert wird.

Für die Durchführung des erfindungsgemäßen Verfahrens ist eine kodierende Gegenstrangnukleinsäure erforderlich. Ein Leseraster im Gegenstrang kann gegebenenfalls durch entsprechende Optimierung der Gegenstrangsequenz erreicht werden. In einer bevorzugten Ausführungsform weist die Gegenstrangnukleinsäure keine internen Stoppkodons auf. Die komplementären Kodons zu den drei Stoppkodons sind relativ selten, daher enthält ein Leseraster im Gegenstrang in der Regel keine Stoppkodons. Vorzugsweise wird die Gegenstrangnukleinsäure gegebenenfalls so optimiert, dass keine internen Stoppkodons enthalten sind.

Eine translationelle Kopplersequenz umfasst erfindungsgemäß ein Stoppkodon, welches *in frame* zu der Gegenstrangnukleinsäure angeordnet ist, und ein darauffolgendes Startkodon, welches *in frame* zu dem Reportergen angeordnet ist. Der Abstand zwischen Stoppkodon und Startkodon ist so gewählt, dass eine translationelle Kopplung ermöglicht wird. Vorzugsweise beträgt der Abstand nicht mehr als 10 Basenpaare.

In einer Ausführungsform der vorliegenden Erfindung schließen Startkodon und Stoppkodon der translationellen Kopplersequenz unmittelbar aneinander an. Beispielsweise kann die translationelle Kopplersequenz zwei getrennte Kodons, z.B. TAA ATG umfassen. In einer anderen Ausführungsform überlappen Startkodon und Stoppkodon der translationellen Kopplersequenz miteinander. Ein Beispiel für eine Überlappung des Stoppkodons mit dem Startkodon ist die translationelle Kopplersequenz TAATG.

Durch die erfindungsgemäße Kopplung der Translation auf genetischer Ebene bei gleichzeitiger Entkopplung der entstehenden Polypeptidketten ist eine signifikante Verbesserung des Verfahren zur Identifikation von korrekten Leserahmen gegeben. Das System bleibt unabhängig von der Faltung der durch die Gegenstrangnukleinsäure gebildeten Polypeptidkette.

In einer bevorzugten Ausführungsform der Erfindung enthält der Linker in den um +1 und -1 verschobenen Leserahmen stromaufwärts des Stoppkodons der translationellen Kopplersequenz gelegene weitere Stoppkodons. Diese Stoppkodons gewährleisten, dass die Translation der verschobenen Leserahmen vor dem Erreichen des Stoppkodons der translationellen Kopplersequenz abgebrochen wird. Der Abstand der weiteren Stoppkodons von dem Startkodon der translationellen Kopplersequenz wird dabei vorzugsweise so gewählt, dass keine translationelle Kopplung erfolgt. Als besonders geeignet hat sich ein Abstand von mindestens 30 Basenpaaren, vorzugsweise mindestens 50 Basenpaaren erwiesen.

Die Vorgehensweise in dem erfindungsgemäßen Verfahren zur Bestimmung von Frameshift-Mutationen entspricht im Übrigen dem Verfahren der DE 10 2007 052 344 B3.

In einer bevorzugten Ausführungsform ist die Gegenstrangnukleinsäure in der Wirtszelle in 3'-Position über einen Linker mit einem Reportergen verknüpft und in 5'-Position operativ mit einer Expressionskontrollsequenz verknüpft.

Eine Expressionskontrollsequenz ist eine Nukleinsäuresequenz, welche die Transkription und Translation kontrolliert und reguliert. Die Expressionskontrollsequenz kann in der Wirtszelle konstitutiv oder regulierbar aktiv sein. Die Formulierung "operativ verknüpft" schließt ein geeignetes Startsignal (z.B. ATG oder AUG) vor der zu exprimierenden Nukleinsäuresequenz und die Beibehaltung des korrekten Leserasters ein, sodass die Expression der Nukleinsäuresequenz unter der Kontrolle der Expressionskontrollsequenz und die Erzeugung des von der Nukleinsäuresequenz kodierten Produktes ermöglicht wird. Wenn eine Nukleinsäure kein geeignetes Startsignal enthält, so kann ein solches Startsignal vor der Nukleinsäure eingefügt werden.

Die Bereitstellung einer Wirtszelle in Schritt (i) des Verfahrens beinhaltet in einer Ausführungsform das Einbringen eines Expressionsvektors in eine Wirtszelle, wobei der Expressionsvektor die doppelsträngige Nukleinsäure umfasst, welche eine kodierende Zielnukleinsäure und eine dazu komplementäre kodierende Gegenstrangnukleinsäure umfasst. In dem Expressionsvektor ist die Gegenstrangnukleinsäure vorzugsweise mit einem Linker in 3'-Position verbunden, welcher die translationelle Kopplersequenz umfasst.

Expressionsvektoren sind dem Fachmann auf dem Gebiet der Molekularbiologie bekannt und beispielsweise in Sambrook et al., Molecular Cloning, A Laboratory Manual (1989), Cold Spring Harbour, Laboratory Press, beschrieben. Als Expressionsvektor kann beispielsweise ein Plasmid oder ein viraler Vektor verwendet werden.

Vorzugsweise ist die Gegenstrangnukleinsäure in dem Expressionsvektor operativ mit einer Expressionskontrollsequenz in 5'-Position verknüpft. In einer besonders bevorzugten Ausführungsform ist die Gegenstrangnukleinsäure in dem Expressionsvektor in 3'-Position über einen Linker mit einem Reportergen verknüpft und in 5'-Position mit einer Expressionskontrollsequenz operativ verknüpft.

Ein Expressionsvektor kann auf beliebige Weise erzeugt werden, beispielsweise durch Kultivierung in Wirtszellen, vorzugsweise in Bakterienzellen wie etwa *E. coli*-Zellen. Zweckmäßigerweise beinhaltet der Expressionsvektor Elemente, die eine Replikation und Selektion in der Wirtszelle ermöglichen. Alternativ kann ein Expressionsvektor auch *in vitro* durch Amplifikation in ausreichender Menge erzeugt werden, beispielsweise durch Polymerase-Kettenreaktion (PCR), Ligase-Kettenreaktion (LCR) oder Rolling-Circle-Amplifikation.

Als Wirtszellen können prokaryotische oder eukaryotische Zellen oder Mikroorganismen verwendet werden. Bei den Wirtszellen kann es sich um Wildtyp-Varianten handeln oder es können mutierte oder genetisch manipulierte Wirtszellen verwendet werden.

Als Reportergen kann ein für ein nachweisbares Genprodukt kodierendes Gen verwendet werden. Beispielsweise kann das Reportergen ein Antibiotika-Resistenzgen, vorzugsweise Kanamycin-Resistenz umfassen. In einer anderen Ausführungsform kann ein Reportergen verwendet werden, welches für ein Fluoreszenzprotein wie etwa GFP kodiert. Weiterhin kann ein Reportergen verwendet werden, das für ein Enzym kodiert, durch das eine colorimetrisch messbare Reaktion katalysiert wird (z.B. β-Galaktosidase). In einer weiteren Ausführungsform kann das Reportergen für ein Genprodukt kodieren, welches mit einer in der Wirtszelle vorhandenen Nukleinsäuresequenz oder mit einem in der Wirtszelle exprimierten Genprodukt wechselwirkt, wodurch es zu einer messbaren Veränderung einer Stoffwechselleistung kommt.

Das Einbringen von Expressionsvektor und gegebenenfalls Reportervektor in die Wirtszelle erfolgt nach im Stand der Technik bekannten Verfahren, beispielsweise durch (Co)Transfektion, (Co)Transformation oder (Co)Infektion von Zellen. Vorzugsweise wird das Verfahren so gewählt, dass die doppelsträngige Nukleinsäure in die Wirtszelle eingebracht wird, sodass in der Wirtszelle die Gegenstrangsequenz über den Linker, welcher eine translationelle Kopplersequenz umfasst, mit dem Reportergen verknüpft ist.

Bei eukaryotischen Wirtszellen kann die Transfektion bzw. Cotransfektion beispielsweise durch Calciumphosphat-Copräzipitation, Lipofektion, Elektroporation, Partikelbeschuss, Verwendung von Bakterienproteinen oder durch virale Infektion über Retroviren, Adenoviren etc. erfolgen.

In einer bevorzugten Ausführungsform wird in Schritt (i) des Verfahrens ein Vektor bereitgestellt, der zusätzlich zu dem über einen Linker mit der Gegenstrangnukleinsäure verknüpften Reportergen mindestens ein Selektionsmarkergen umfasst. Das mindestens eine Selektionsmarkergen ist ausgewählt aus beliebigen für ein nachweisbares Genprodukt kodierenden Genen, wobei das Genprodukt vorzugsweise von dem Genprodukt des Reportergens verschieden ist. Beispielsweise kann das Selektionsmarkergen ein Antibiotikaresistenzgen wie etwa β-Lactamase für Ampicilinresistenz umfassen. Alternativ kann als Selektionsmarkergen ein für ein Fluoreszenzprotein wie etwa GFP kodierendes Gen verwendet werden.

Über das Selektionsmarkergen kann unabhängig von dem Reportergen eine Selektion erfolgen. Beispielsweise kann ein Konstrukt nach erfolgter Selektion auf das erste Reportergen (beispielsweise auf Kanamycinresistenz) über Selektion mittels des weiteren Selektionsmarkergens (z.B. für Ampicillinresistenz) weiter hochamplifiziert werden. Darüber hinaus besteht die Möglichkeit, Konstrukte die eine Frameshift-Mutation enthalten (z.B. eine absichtliche Frameshift-Mutation) durch Selektion über das zweite Selektionsmarkergen weiter zu amplifizieren.

Schließlich kann das zweite Selektionsmarkergen dazu beitragen, die Existenz des Reportergens im Zusammenhang mit einer stattgefundenen Selektion zu verschleiern, um so das Verfahren vor Nachahmung zu schützen.

Schritt (ii) des Verfahrens beinhaltet das Bewirken der Expression der Gegenstrangnukleinsäure in der Wirtszelle. Hierzu wird die Wirtszelle unter geeigneten Bedingungen kultiviert, die eine Expression der Gegenstrangnukleinsäure erlauben.

Schritt (iii) beinhaltet die Bestimmung, ob in der Wirtszelle eine Expression des Reportergens erfolgt. Die Bestimmung beruht darauf, dass ein von dem Reportervektor codiertes Genprodukt nachgewiesen wird. Die Bestimmung kann abhängig von dem verwendeten Reportergen grundsätzlich die Bestimmung beliebiger phänotypisch erkennbarer Effekte umfassen, beispielsweise morphologische Veränderungen, Änderungen des Wachstumsverhaltens, etc, Wenn das Reportergen beispielsweise ein Fluoreszenzprotein wie GFP kodiert, so kann das Vorhandensein und/oder die Intensität der Lumineszenz z.B. mittels Fluoreszenzcytometrie oder Imaging-Assays bestimmt werden. Wenn als Reportergen ein Antibiotika-Resistenzgen verwendet wird, so kann die Bestimmung auf Grundlage des Wachstums von Wirtszellen in Gegenwart von Antibiotika erfolgen.

In einer Ausführungsform kann eine Zielnukleinsäure aus zwei, drei oder mehreren Teilfragmenten zusammengesetzt sein. Beispielsweise können bei einem Konstrukt mit 3 kB zunächst drei Teilfragmente aus Oligonukleotiden hergestellt werden und diese Teilfragmente dann z.B. über Fusions-PCR zu einem 3 kB-Fragment fusioniert werden. Ein so fusioniertes Konstrukt aus zwei oder mehreren Teilfragmenten kann dann direkt in einen Expressionsvektor ligiert werden.

Da mit dem Verfahren Frameshift-Mutationen, aber keine Mutationen unter Erhalt des Leserasters bestimmt werden können, wird bei der Synthese der Zielnukleinsäure durch PCR vorzugsweise der Amplifikationsprimer zu einem möglichst späten Zeitpunkt zugegeben, um die Anzahl der Amplifikationsschritte möglichst klein zu halten. Je höher die Anzahl amplifizierbarer Volllängenmoleküle, desto weniger durch PCR zustande gekommene Mutationen sind im PCR-Produkt enthalten.

Um Substitutionen noch weiter zu verringern, kann beispielsweise Ofloxazin nach der Transformation eingesetzt werden. Ofloxazin ist ein Gyrasehemmer, der die DNA-Replikation hemmt, Wirtszellen wie *E*. *coli-*Zellen jedoch kurzfristig nicht tötet. Während dieses Zeitraums besteht für eine Wirtszelle die Möglichkeit, Heterodimere über einen internen Reparaturmechanismus zu spalten. Vorzugsweise kann Ofloxazin im Medium nach der Elektroporation oder Hitzeschocktransformation enthalten sein (0,5-1 h bei 37 °C). Im Anschluss werden die Zellen abzentrifugiert, das Medium zusammen mit dem Ofloxazin entfernt und die Zellen werden ausplattiert.

### Figuren

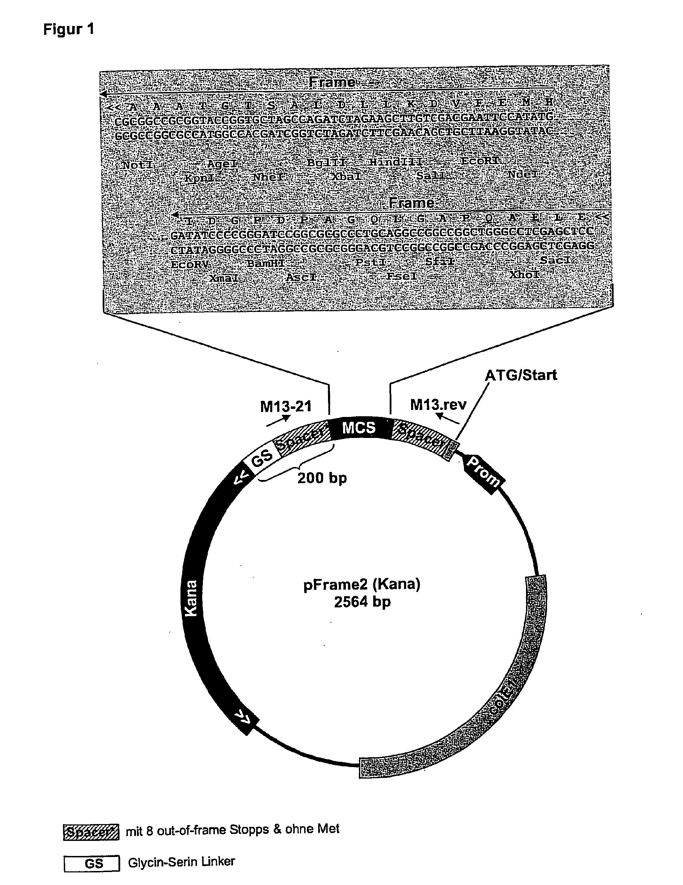

**Figur 1** zeigt die Plasmidkarte eines Expressionsvektors gemäß DE 10 2007 052 344 B3. Als Reportergen ist Kanamycin-Resistenz stromabwärts der Gegenstrangnukleinsäure MCS enthalten.

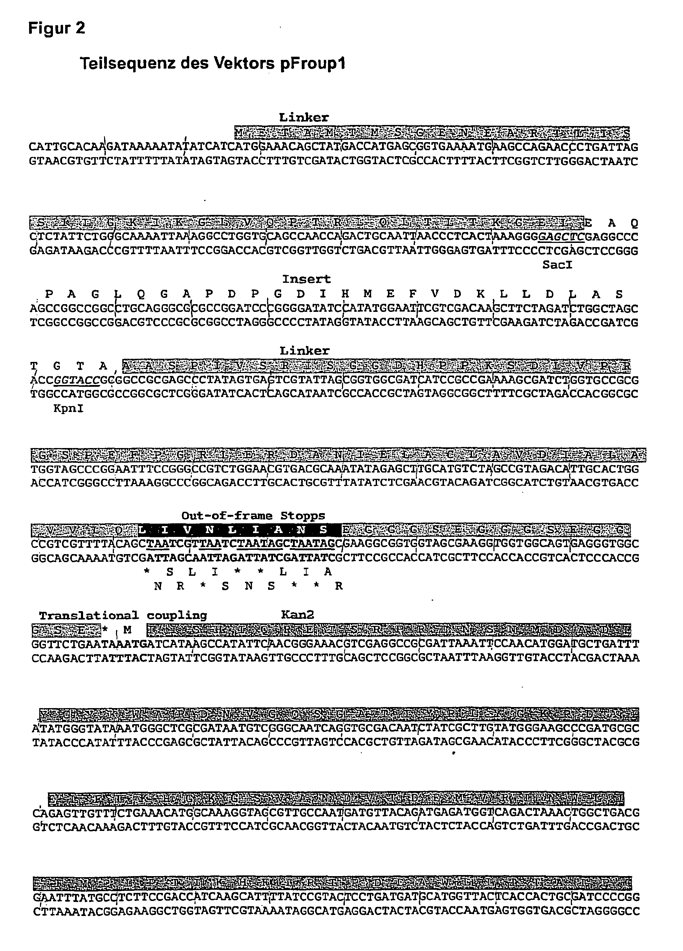

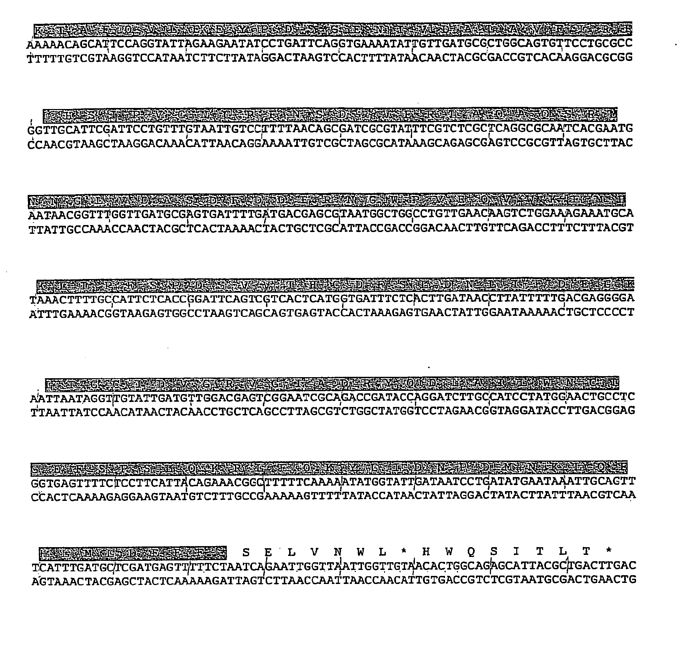

**Figur 2** zeigt eine Teilsequenz eines Vektors pFroup1 zur Verwendung in dem Verfahren der Erfindung. Die Gegenstrangnukleinsäure (Insert) wurde über einen Linker mit einem Kanamycinresistenzgen (kan2) verknüpft. Der gezeigte Linker enthält in den zu dem Leserahmen der Gegenstrangsequenz um +1 und um -1 verschobenen Leserahmen jeweils mehrere Stoppkodons. Weiter enthält der Linker eine translationelle Kopplersequenz (TAA ATG), deren Stoppkodon *in frame* zu dem Leserahmen der Gegenstrangnukleinsäure liegt, und deren Startkodon *in frame* zu dem Kanamycinresistenzgen liegt.

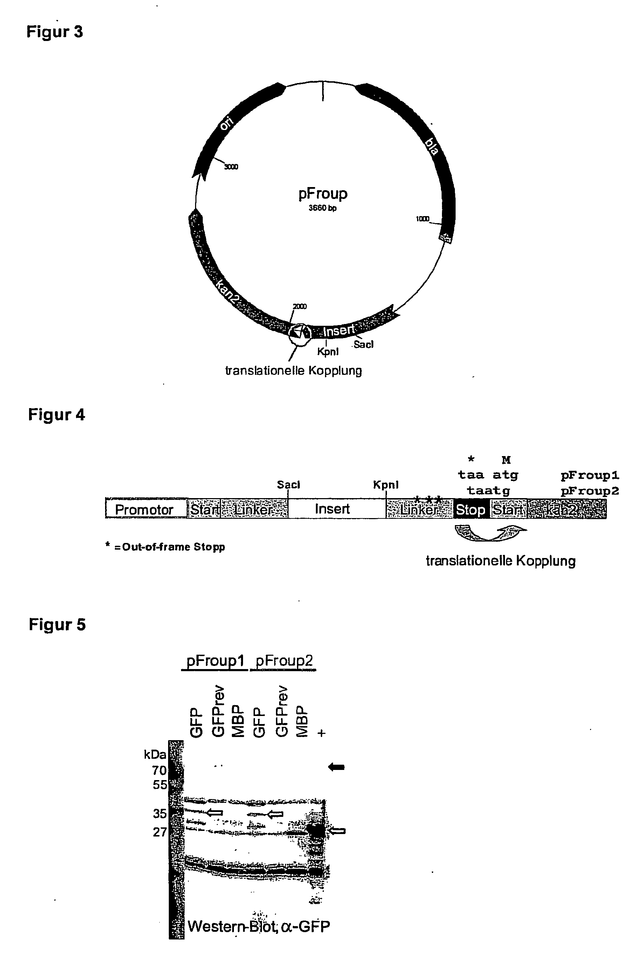

**Figur 3** zeigt den Aufbau der erfindungsgemäßen Vektoren pFroup1 und pFroup2. Beide sind für die Verwendung in E.coli konzipiert. Das Plasmid verleiht der Wirtszelle eine Ampicilinresistenz, unabhängig von einem Insert, so dass eine problemlose Vermehrung sichergestellt ist. Das zu untersuchende Insert wird über die Schnittstellen Kpnl und Sacl in den Vektor kloniert. Die Expression steht unter der Kontrolle eines konstitutiven Promotors.
**Figur 4** ist eine Darstellung der Kopplung von Insert und kan2. N-terminal wird an das Insert ein Linker fusioniert, der im +1 und -1 Leserahmen Stopps trägt (out-of-frame Stopps). Bei Deletionen und Insertionen, die den Leserahmen verschieben, wird die Translation an dieser Stelle abgebrochen. Nur bei korrekter Translation des Inserts erfolgt eine Synthese des Resistenzmarkers über translationelle Kopplung, die in diesem Fall zur Kanamycinresistenz der Wirtszelle führt.
**Figur 5** zeigt das Ergebnis einer Immunoblot-Analyse des Expressionsprodukts einer für GFP kodierenden Nukleinsäuresequenz, die in die Plasmide pFroup1 und 2 eingefügt wurde. Das in den transformierten Zellen synthetisierte GFP wurde mittels Immunoblot untersucht, um nachzuweisen, dass über die translationelle Kopplung nicht ein großes Fusionsprotein gebildet wird, sondern zwei getrennte Polypeptidketten entstehen. Als Kontrolle dienten Zellen, die mit GFPrev, der Gegenstrangnukleinsäure von GFP, welche für ein nonsense-Polypeptid kodiert, oder MBP transformiert wurden. Freies GFP hat eine Größe von 40 kDa (weißer Pfeil), das GFP-Kan2 Fusionsprotein wäre 70 kDa groß (schwarzer Pfeil). Als Positivkontrolle (+) ist GFP aus einem Vergleichsvektor aufgetragen. Da hier N-terminal kein Linker vorhanden ist, ist es nur ca. 30 kDa groß. Sowohl in pFroup1 als auch in pFroup2 funktioniert die translationelle Kopplung. Somit wird kein detektierbares Kan2 Fusionsprotein gebildet.

Die Erfindung soll durch das nachstehende Ausführungsbeispiel weiter veranschaulicht werden.

### Beispiel

Es wurden die in **Fig. 2-4** veranschaulichten Vektoren pFroup1 und 2 synthetisiert, in denen eine zu untersuchende Nukleinsäure (Insert) über einen Linker, der eine translationelle Kopplersequenz umfasst, mit einem Reportergen verknüpft ist. Die Vektoren unterscheiden sich in der Art der translationellen Kopplung. Bei pFroup1 bestehen Stopp und Start aus zwei getrennten Kodons (TAA ATG). Bei pFroup2 überlappt das Stoppkodon mit dem Startkodon (TAATG). An der Position des Inserts kann eine beliebige, auf Frameshift-Mutationen zu überprüfende Nukleinsäure eingefügt werden.

Zur Überprüfung der Funktionsfähigkeit der erfindungsgemäßen Vektoren wurden zunächst gezielt Modell-Inserts getestet. Dabei wurde eine für das grün fluoreszierende Protein (GFP) kodierende Nukleinsäuresequenz, die dazu revers komplementäre Sequenz (GFPrev) und ein Teil des Maltose bindenden Proteins (MBP) als Insert in pFroup1 und 2 eingefügt. Die Sequenzen GFP und GFPrev haben ein korrektes Leseraster, das Insert MBP hat keine durch drei teilbare Sequenz, also keinen korrekten Leserahmen.

Die Funktionsweise der Vektoren wurde zunächst im Wachstumstest gezeigt. *E. coli*-Wirtszellen, welche die Vektoren pFroup-GFP und pFroup-GFPrev enthalten, zeigten Resistenz gegenüber Ampicillin und Kanamycin. Der Vektor pFroup-MBP verleiht der Wirtszelle keine Kanamycin-Resistenz, da hier aufgrund des inkorrekten Leserahmens durch den vorzeitigen Abbruch der Translation im Linkerbereich das Polypeptid Kan2 nicht gebildet wird. Um auszuschließen, dass die Kanamycinresistenz des Konstrukts pFroup-GFP auf der Ausbildung eines Fusionsproteins aus GFP und Kan2 beruht (das könnte in pFroup1 durch Überlesen des Stopp-Codons geschehen), wurde das synthetisierte GFP mittels Immunoblot-Analyse untersucht **(****Fig.5****)**. Es konnte das freie GFP, jedoch nicht ein sehr viel größeres GFP-Kan2 Fusionsprotein detektiert werden. Somit war eine korrekte Funktionsweise der translationellen Kopplung in den entwickelten Vektoren pFroup 1 und 2 gegeben.

## Patentansprüche

1. Verfahren zur Bestimmung von Frameshift-Mutationen in kodierenden Zielnukleinsäuren, welches die Schritte umfasst:
(i) Bereitstellen einer Wirtszelle, umfassend eine doppelsträngige Nukleinsäure, welche eine kodierende Zielnukleinsäure und eine dazu komplementäre, kodierende Gegenstrangnukleinsäure umfasst, worin die Gegenstrangnukleinsäure über einen Linker mit einem Reportergen in 3'-Position verknüpft ist;
(ii) Bewirken der Expression der Gegenstrangnukleinsäure; und
(iii) Bestimmen, ob in der Wirtszelle eine Expression des Reportergens erfolgt,
worin die Expression des Reportergens anzeigt, dass die Zielnukleinsäure keine Frameshift-Mutation aufweist,
**dadurch gekennzeichnet,**
**dass** der Linker eine translationelle Kopplersequenz aufweist, die ein Stoppkodon *in frame* zu dem Leserahmen der Gegenstrangnukleinsäure und ein Startkodon umfasst, wobei das Reportergen *in frame* zu dem Startkodon liegt.

2. Verfahren nach Anspruch 1,
wobei der Linker in den um +1 und -1 verschobenen Leserahmen stromaufwärts des Stoppkodons der translationellen Koppler-Sequenz gelegene weitere Stoppkodons aufweist.

3. Verfahren nach Anspruch 2,
wobei der Abstand von den weiteren Stoppkodons zu dem Startkodon der translationellen Kopplersequenz so gewählt ist, dass keine translationelle Kopplung erfolgt, vorzugsweise mindestens 30 Basenpaare , insbesondere mindestens 50 Basenpaare.

4. Verfahren nach einem der Ansprüche 1 bis 3,
wobei Start- und Stoppkodon der translationellen Kopplersequenz unmittelbar aneinander anschließen.

5. Verfahren nach einem der Ansprüche 1 bis 3,
wobei Start- und Stoppkodon der translationellen Kopplersequenz miteinander überlappen.

6. Verfahren nach einem der Ansprüche 1 bis 3,
wobei Start- und Stoppkodon der translationellen Kopplersequenz einen Abstand voneinander aufweisen, der so gewählt ist, dass eine translationelle Kopplung ermöglicht wird, vorzugsweise einen Abstand von nicht mehr als 10 Basenpaaren.

7. Verfahren nach einem der Ansprüche 1 bis 6,
worin die Gegenstrangnukleinsäure weiterhin in operativer Verknüpfung mit einer Expressionskontrollsequenz in 5'-Position vorliegt.

8. Verfahren nach einem der Ansprüche 1 bis 7,
worin Schritt (i) umfasst:
- Einbringen eines Expressionsvektors, umfassend die doppelsträngige Nukleinsäure, welche eine kodierende Zielnukleinsäure und eine dazu komplementäre, kodierende Gegenstrangnukleinsäure umfasst, in eine Wirtszelle, wobei die Gegenstrangnukleinsäure in dem Expressionsvektor über einen Linker mit einem Reportergen in 3'-Position verknüpft ist.

9. Verfahren nach Anspruch 8, worin die Gegenstrangnukleinsäure in dem Expressionsvektor in operativer Verknüpfung mit einer Expressionskontrollsequenz in 5' Position vorliegt.

10. Verfahren nach einem der Ansprüche 8 oder 9, worin in Schritt (i) ein Expressionsvektor verwendet wird, der zusätzlich mindestens ein Selektionsmarkergen umfasst.

11. Verfahren nach Anspruch 10, worin das Selektionsmarkergen konstitutiv exprimiert wird.

12. Verfahren nach einem der vorhergehenden Ansprüche, worin die Gegenstrangnukleinsäure so optimiert wird, dass sie keine StoppKodons umfasst.

13. Verfahren nach einem der vorhergehenden Ansprüche, worin die Nukleinsäuresequenz aus genomischen Sequenzen, cDNA-Sequenzen, oder cDNA-Fragmenten stammt.

14. Verfahren nach einem der vorhergehenden Ansprüche, worin die Nukleinsäuresequenz aus einer Bibliothek, die eine Kollektion von Sequenzen enthält, stammt.

15. Verfahren nach einem der vorhergehenden Ansprüche, worin die Zielnukleinsäuresequenz aus einer normalisierten cDNA-Bibliothek stammt.

16. Verfahren nach einem der vorhergehenden Ansprüche, worin als Expressionsvektor ein Plasmid verwendet wird.

17. Verfahren nach einem der vorhergehenden Ansprüche, worin als Wirtszelle eine eukaryotische Zelle verwendet werden.

18. Verfahren nach einem der Ansprüche 8 bis 17, worin das Einbringen des Expressionsvektors durch Calciumphosphat-Copräzipitation, Lipofektion, Elektroporation, Partikelbeschuss oder virale Infektion erfolgt.

19. Verfahren nach einem der vorhergehenden Ansprüche, worin das Reportergen ein für ein nachweisbares Genprodukt codierendes Gen enthält.

20. Verfahren nach Anspruch 19, worin das Reportergen ein Antibiotika-Resistenzgen wie etwa Kanamycin-Resistenz umfasst.

21. Verfahren nach Anspruch 19, worin das nachweisbare Genprodukt aus Fluoreszenzproteinen wie vorzugsweise GFP ausgewählt wird.

22. Verfahren nach Anspruch 19, worin das nachweisbare Genprodukt eine colorimetrisch messbare Reaktion katalysiert.

23. Verfahren nach einem der vorhergehenden Ansprüche, worin die Bestimmung in Schritt (iii) durch Fluoreszenzcytometrie oder Imaging-Assays erfolgt.

## Claims

1. Method for determining frameshift mutations in coding target nucleic acids, comprising the steps:
(i) making available a host cell, comprising a double-stranded nucleic acid, which comprises a coding target nucleic acid and a coding counter-strand nucleic acid that is complementary to it, wherein the counter-strand nucleic acid is linked with a reporter gene in the 3' position, by way of a linker;
(ii) bringing about expression of the counter-strand nucleic acid; and
(iii) determining whether expression of the reporter gene takes place in the host cell,
wherein expression of the reporter cell indicates that the target nucleic acid does not have any frameshift mutation,
**characterized in that**
the linker has a translational coupler sequence that comprises a stop codon *in frame* to the reading frame of the counter-strand nucleic acid, and a start codon, where the reporter gene lies *in frame* to the start codon.

2. Method according to claim 1,
where the linker has additional stop codons that lie upstream from the stop codon of the translational coupler sequence, in the read frame shifted by +1 and -1.

3. Method according to claim 2,
where the distance of the additional stop codons from the start codon of the translational coupler sequence is selected in such a manner that no translational coupling takes place, preferably at least 30 base pairs, particularly at least 50 base pairs.

4. Method according to one of claims 1 to 3,
where the start codon and stop codon of the translational coupler sequence fellow one another directly.

5. Method according to one of claims 1 to 3,
where the start codon and stop codon of the translational coupler sequence overlap one another.

6. Method according to one of claims 1 to 3,
where the start codon and stop codon of the translational coupler sequence have a distance from one another that is selected in such a manner that translational coupling is made possible, preferably a distance of not more than 10 base pairs.

7. Method according to one of claims 1 to 6,
wherein the counter-strand nucleic acid continues to be present in an operative link with an expression control sequence in the 5' position.

8. Method according to one of claims 1 to 7,
wherein step (i) comprises:
- introducing an expression vector, comprising the double-stranded nucleic acid, which comprises a coding target nucleic acid and a coding counter-strand nucleic acid that is complementary to it, into a host cell, where the counter-strand nucleic acid in the expression vector is linked with a reporter gene in the 3' position, by way of a linker.

9. Method according to claim 8, wherein the counter-strand nucleic acid in the expression vector is present in an operative link with an expression control sequence in the 5' position.

10. Method according to one of claims 8 or 9, wherein in step (i), an expression vector is used that additionally comprises at least one selection marker gene.

11. Method according to claim 10, wherein the selection marker gene is expressed constitutively.

12. Method according to one of the preceding claims, wherein the counter-strand nucleic acid is optimized in such a manner that it does not comprise any stop codons.

13. Method according to one of the preceding claims, wherein the nucleic acid sequence is derived from genomic sequences, cDNA sequences, or cDNA fragments.

14. Method according to one of the preceding claims, wherein the nucleic acid sequence is derived from a library that contains a collection of sequences.

15. Method according to one of the preceding claims, wherein the target nucleic acid sequence is derived from a normalized cDNA library.

16. Method according to one of the preceding claims, wherein a plasmid is used as an expression vector.

17. Method according to one of the preceding claims, wherein a eukaryotic cell are used [sic - plural verb] as the host cell.

18. Method according to one of claims 8 to 17, wherein the introduction of the expression vector takes place by means of calcium phosphate coprecipitation, lipofection, electroporation, particle bombardment, or viral infection.

19. Method according to one of the preceding claims, wherein the reporter gene contains a gene that codes for a detectable gene product.

20. Method according to claim 19, wherein the reporter gene comprises an antibiotic resistance gene such as kanamycin resistance, for example.

21. Method according to claim 19, wherein the detectable gene product is selected from among fluorescence proteins such as preferably GFP.

22. Method according to claim 19, wherein the detectable gene product catalyzes a reaction that can be measured by colorimetry.

23. Method according to one of the preceding claims, wherein the determination in step (iii) takes place by means of fluorescence cytometry or imaging assays.

## Revendications

1. Procédé de détermination de mutations à décalage du cadre de lecture dans des acides nucléiques cibles codants, lequel comprend les étapes de :
**(i)** préparation d'une cellule hôte, comprenant un acide nucléique double brin, lequel comprend un acide nucléique cible codant et un acide nucléique de contre-brin codant, complémentaire de celui-ci, l'acide nucléique de contre-brin étant lié en position 3' à un gène rapporteur par l'intermédiaire d'une séquence de liaison ;
**(ii)** obtention de l'expression de l'acide nucléique de contre-brin ; et
**(iii)** détermination du point de savoir si une expression du gène rapporteur a lieu dans la cellule hôte,
où l'expression du gène rapporteur indique que l'acide nucléique cible ne présente pas de mutation à décalage du cadre de lecture,
**caractérisé par le fait que** la séquence de liaison comprend une séquence de couplage traductionnel, qui comprend un codon d'arrêt, *dans le cadre* par rapport au cadre de lecture de l'acide nucléique de contre-brin, et un codon d'initiation, le gène rapporteur se situant *dans le cadre* par rapport au codon d'initiation.

2. Procédé selon la revendication 1,
suivant lequel la séquence de liaison comprend d'autres codons d'arrêt situés dans le cadre de lecture décalé de +1 et -1, en amont du codon d'arrêt de la séquence de couplage traductionnel.

3. Procédé selon la revendication 2,
dans lequel la distance entre les autres codons d'arrêt et le codon d'initiation de la séquence de couplage traductionnel est choisie de telle sorte qu'aucun couplage traductionnel n'a lieu, de préférence au moins 30 paires de bases, en particulier au moins 50 paires de bases.

4. Procédé selon l'une des revendications 1 à 3, dans lequel le codon d'initiation et le codon d'arrêt de la séquence de couplage traductionnel se suivent sans intermédiaire.

5. Procédé selon l'une des revendications 1 à 3, dans lequel le codon d'initiation et le codon d'arrêt de la séquence de couplage traductionnel se chevauchent l'un l'autre.

6. Procédé selon l'une des revendications 1 à 3, dans lequel le codon d'initiation et le codon d'arrêt de la séquence de couplage traductionnel sont espacés l'un de l'autre d'une distance qui est choisie de telle sorte qu'un couplage traductionnel est rendu possible, de préférence une distance de pas plus de 10 paires de bases.

7. Procédé selon l'une des revendications 1 à 6, dans lequel l'acide nucléique de contre-brin est en outre présent en liaison fonctionnelle avec une séquence de contrôle d'expression en position 5'.

8. Procédé selon l'une des revendications 1 à 7, dans lequel l'étape (i) comprend :
- amener un vecteur d'expression, comportant l'acide nucléique double brin, lequel comprend un acide nucléique cible codant et un acide nucléique de contre-brin codant, complémentaire de celui-ci, dans une cellule hôte, l'acide nucléique de contre-brin étant lié en position 3', dans le vecteur d'expression, par l'intermédiaire d'une séquence de liaison ayant un gène rapporteur.

9. Procédé selon la revendication 8, dans lequel l'acide nucléique de contre-brin est présent, dans le vecteur d'expression, en liaison fonctionnelle en position 5' avec une séquence de contrôle d'expression.

10. Procédé selon l'une des revendications 8 ou 9, dans lequel, à l'étape (i), on utilise un vecteur d'expression qui comprend en outre au moins un gène marqueur de sélection.

11. Procédé selon la revendication 10, dans lequel le gène marqueur de sélection est exprimé de façon constitutive.

12. Procédé selon l'une des revendications précédentes, dans lequel l'acide nucléique de contre-brin est optimisé de telle sorte qu'il ne comprend pas de codon d'arrêt.

13. Procédé selon l'une des revendications précédentes, dans lequel la séquence d'acide nucléique provient de séquences génomiques, de séquences d'ADNc ou de fragments d'ADNc.

14. Procédé selon l'une des revendications précédentes, dans lequel la séquence d'acide nucléique provient d'une bibliothèque qui contient une collection de séquences.

15. Procédé selon l'une des revendications précédentes, dans lequel la séquence d'acide nucléique cible provient d'une bibliothèque normalisée d'ADNc.

16. Procédé selon l'une des revendications précédentes, dans lequel on utilise un plasmide comme vecteur d'expression.

17. Procédé selon l'une des revendications précédentes, dans lequel on utilise une cellule eucaryote comme cellule hôte.

18. Procédé selon l'une des revendications 8 à 17, dans lequel l'introduction du vecteur d'expression a lieu par co-précipitation avec du phosphate de calcium, lipofection, électroporation, bombardement de particules ou infection virale.

19. Procédé selon l'une des revendications précédentes, dans lequel le gène rapporteur contient un gène codant pour un produit génique détectable.

20. Procédé selon la revendication 19, dans lequel le gène rapporteur comprend un gène de résistance aux antibiotiques comme par exemple la résistance à la kanamycine.

21. Procédé selon la revendication 19, dans lequel le produit génique détectable est choisi parmi les protéines fluorescentes comme par exemple la GFP.

22. Procédé selon la revendication 19, dans lequel le produit génique détectable catalyse une réaction mesurable par colorimétrie.

23. Procédé selon l'une des revendications précédentes, dans lequel la détermination à l'étape (iii) a lieu par cytométrie par fluorescence ou analyse par imagerie.


### SEQUENCE LISTING

<110> GENEART AG
<120> Selektion kodierender Nukleinsäure-Konstrukte auf Abwesenheit von Frameshift-Mutationen
<130> 40860P DE-1
<140> DE 10 2009 011 253.7-41
   <141> 2009-11-23
<160> 2
<170> PatentIn version 3.3
<210> 1
   <211> 1360
   <212> DNA
   <213> Artificial
<220>
   <223> Teilsequenz des Vektors pFroup1
<400> 1
   cattgcacaa gataaaaata tatcatcatg gaaacagcta tgaccatgag cggtgaaaat 60
   gaagccagaa ccctgattag ctctattctg ggcaaaatta aaggcctggt gcagccaacc 120
   agactgcaat taaccctcac taaaggggag ctcgaggccc agccggccgg cctgcagggc 180
   gcgccggatc ccggggatat ccatatggaa ttcgtcgaca agcttctaga tctggctagc 240
   accggtaccg cggccgcgag ccctatagtg agtcgtatta gcggtggcga tcatccgccg 300
   aaaagcgatc tggtgccgcg tggtagcccg gaatttccgg gccgtctgga acgtgacgca 360
   aatatagagc ttgcatgtct agccgtagac attgcactgg ccgtcgtttt acagctaatc 420
   gttaatctaa tagctaatag cgaaggcggt ggtagcgaag gtggtggcag tgagggtggc 480
   ggttctgaat aaatgatcat aagccatatt caacgggaaa cgtcgaggcc gcgattaaat 540
   tccaacatgg atgctgattt atatgggtat aaatgggctc gcgataatgt cgggcaatca 600
   ggtgcgacaa tctatcgctt gtatgggaag cccgatgcgc cagagttgtt tctgaaacat 660
   ggcaaaggta gcgttgccaa tgatgttaca gatgagatgg tcagactaaa ctggctgacg 720
   gaatttatgc ctcttccgac catcaagcat tttatccgta ctcctgatga tgcatggtta 780
   ctcaccactg cgatccccgg aaaaacagca ttccaggtat tagaagaata tcctgattca 840
   ggtgaaaata ttgttgatgc gctggcagtg ttcctgcgcc ggttgcattc gattcctgtt 900
   tgtaattgtc cttttaacag cgatcgcgta tttcgtctcg ctcaggcgca atcacgaatg 960
   aataacggtt tggttgatgc gagtgatttt gatgacgagc gtaatggctg gcctgttgaa 1020
   caagtctgga aagaaatgca taaacttttg ccattctcac cggattcagt cgtcactcat 1080
   ggtgatttct cacttgataa ccttattttt gacgagggga aattaatagg ttgtattgat 1140
   gttggacgag tcggaatcgc agaccgatac caggatcttg ccatcctatg gaactgcctc 1200
   ggtgagtttt ctccttcatt acagaaacgg ctttttcaaa aatatggtat tgataatcct 1260
   gatatgaata aattgcagtt tcatttgatg ctcgatgagt ttttctaatc agaattggtt 1320
   aattggttgt aacactggca gagcattacg ctgacttgac 1360
<210> 2
   <211> 440
   <212> PRT
   <213> Artificial
<220>
   <223> Teilsequenz des Vektors pFroup1
<400> 2
   Met Glu Thr Ala Met Thr Met Ser Gly Glu Asn Glu Ala Arg Thr Leu
   1               5                   10                  15
   Ile Ser Ser Ile Leu Gly Lys Ile Lys Gly Leu Val Gln Pro Thr Arg
               20                  25                  30
   Leu Gln Leu Thr Leu Thr Lys Gly Glu Leu Glu Ala Gln Pro Ala Gly
           35                  40                  45
   Leu Gln Gly Ala Pro Asp Pro Gly Asp Ile His Met Glu Phe Val Asp
       50                  55                  60
   Lys Leu Leu Asp Leu Ala Ser Thr Gly Thr Ala Ala Ala Ser Pro Ile
   65                  70                  75                  80
   Val Ser Arg Ile Ser Gly Gly Asp His Pro Pro Lys Ser Asp Leu Val
                   85                  90                  95
   Pro Arg Gly Ser Pro Glu Phe Pro Gly Arg Leu Glu Arg Asp Ala Asn
               100                 105                 110
   Ile Glu Leu Ala Cys Leu Ala Val Asp Ile Ala Leu Ala Val Val Leu
           115                 120                 125
   Gln Leu Ile Val Asn Leu Ile Ala Asn Ser Glu Gly Gly Gly Ser Glu
       130                 135                 140
   Gly Gly Gly Ser Glu Gly Gly Gly Ser Glu Met Ile Ile Ser His Ile
   145                 150                 155                 160
   Gln Arg Glu Thr Ser Arg Pro Arg Leu Asn Ser Asn Met Asp Ala Asp
                   165                 170                 175
   Leu Tyr Gly Tyr Lys Trp Ala Arg Asp Asn Val Gly Gln Ser Gly Ala
               180                 185                 190
   Thr Ile Tyr Arg Leu Tyr Gly Lys Pro Asp Ala Pro Glu Leu Phe Leu
           195                 200                 205
   Lys His Gly Lys Gly Ser Val Ala Asn Asp Val Thr Asp Glu Met Val
       210                 215                 220
   Arg Leu Asn Trp Leu Thr Glu Phe Met Pro Leu Pro Thr Ile Lys His
   225                 230                 235                 240
   Phe Ile Arg Thr Pro Asp Asp Ala Trp Leu Leu Thr Thr Ala Ile Pro
                   245                 250                 255
   Gly Lys Thr Ala Phe Gln Val Leu Glu Glu Tyr Pro Asp Ser Gly Glu
               260                 265                 270
   Asn Ile Val Asp Ala Leu Ala Val Phe Leu Arg Arg Leu His Ser Ile
           275                 280                 285
   Pro Val Cys Asn Cys Pro Phe Asn Ser Asp Arg Val Phe Arg Leu Ala
       290                 295                 300
   Gln Ala Gln Ser Arg Met Asn Asn Gly Leu Val Asp Ala Ser Asp Phe
   305                 310                 315                 320
   Asp Asp Glu Arg Asn Gly Trp Pro Val Glu Gln Val Trp Lys Glu Met
                   325                 330                 335
   His Lys Leu Leu Pro Phe Ser Pro Asp Ser Val Val Thr His Gly Asp
               340                 345                 350
   Phe Ser Leu Asp Asn Leu Ile Phe Asp Glu Gly Lys Leu Ile Gly Cys
           355                 360                 365
   Ile Asp Val Gly Arg Val Gly Ile Ala Asp Arg Tyr Gln Asp Leu Ala
       370                 375                 380
   Ile Leu Trp Asn Cys Leu Gly Glu Phe Ser Pro Ser Leu Gln Lys Arg
   385                 390                 395                 400
   Leu Phe Gln Lys Tyr Gly Ile Asp Asn Pro Asp Met Asn Lys Leu Gln
                   405                 410                 415
   Phe His Leu Met Leu Asp Glu Phe Phe Ser Glu Leu Val Asn Trp Leu
               420                 425                 430
   His Trp Gln Ser Ile Thr Leu Thr
           435                 440